# FASCICULE DE BREVET EUROPEEN

(11) **EP 3 201 247 B1**
(45) Date de publication et mention de la délivrance du brevet: **27.02.2019**
(21) Numéro de dépôt: 15778900.9
(22) Date de dépôt: 29.09.2015
(51) Int. Cl.: C08F 220/18, A61Q 19/08, A61K 8/81

(54) **POLYMÈRE ACRYLIQUE D'ANHYDRIDE MALÉIQUE ET SON UTILISATION EN COSMÉTIQUE**
ACRYLPOLYMER VON MALEINSÄUREANHYDRID UND VERWENDUNG DAVON IN KOSMETIKA
ACRYLIC POLYMER OF MALEIC ANHYDRIDE AND USE THEREOF IN COSMETICS

(30) Priorité: 30.09.2014 FR 1459232
(43) Date de publication de la demande: 09.08.2017
(73) Titulaire: L'OREAL, 75008 Paris (FR)
(72) Inventeur: SCHULTZE, Xavier, F-93320 Les Pavillons sous Bois (FR); HERNANDEZ, Franck, F-93250 Villemomble (FR); LION, Bertrand, 93601 Aulnay-Sous-Bois (FR)
(74) Mandataire: Kromer, Christophe
(86) Numéro de dépôt international: PCT/EP2015/072460
(87) Numéro de publication internationale: WO 2016/050785

(56) Documents cités:
- FR-A1- 2 972 630
- US-A- 5 185 143
- US-A1- 2014 227 210

## Description

La présente invention concerne un polymère acrylique d'anhydride maléique, une composition comprenant un tel polymère et l'utilisation de ce polymère dans le domaine cosmétique.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge.

Il est connu de traiter ces signes du vieillissement en utilisant des compositions cosmétiques ou dermatologiques contenant des actifs capables de lutter contre le vieillissement, tels que les α-hydroxy-acides, les β-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

Les inventeurs ont découvert qu'un polymère acrylique d'anhydride maléique particulier tel que décrit ci-après présente de bonnes propriétés filmogènes. Lorsque le polymère est appliqué sur la peau, il présente un bon effet tenseur sur la peau et permet ainsi d'atténuer les rides de la peau de façon immédiate. Cet effet tenseur présente également une bonne résistance à l'eau. Ce polymère acrylique particulier est facilement véhiculable dans une huile hydrocarbonée comme l'isododécane. Ce polymère est donc bien adapté pour réaliser des compositions anhydres à effet tenseur ayant une bonne résistance à l'eau. Le film obtenu avec le polymère présente en outre une bonne résistance à l'eau.

Ces polymères filmogènes conviennent également pour le maquillage de la peau ou des lèvres tels que les fonds de teint, les rouges à lèvres.

US 2014/227210 décrit des copolymères comprenant au moins deux monomères choisis parmi le (méth)acrylate d'isobornyle, le (méth)acrylate d'isobutyle et l'acrylate d'éthyle-2-hexyle. Le copolymère peut contenir de 0 à 50 % en poids d'un troisième monomère. Les polymères sont utilisés en cosmétique.

US5185143 décrit un terpolymère anhydride maléique / acétate de vinyle/ (méth)acrylate d'isobornyle selon un ratio molaire 0,35-1 /1/0,05-0,25 ; l'acétate de vinyle y est présent en une quantité massique plus importante que le (méth)acrylate d'isobornyle.

De façon plus précise, la présente invention a pour objet un copolymère issu de la polymérisation de :
(a) 50 à 80 % en poids, du poids total de monomères, de (méth)acrylate d'isobornyle
(b) 5 à 30 % en poids d'anhydride maléique
(c) 15 à 30 % en poids de monomère acrylate d'alkyle en C₆-C₁₆.

Un tel copolymère est appelé par la suite polymère acrylique.

L'invention a également pour objet une composition comprenant, dans un milieu physiologiquement acceptable, un polymère acrylique tel que décrit précédemment.

L'invention a également pour objet un procédé, notamment cosmétique, de soin ou de maquillage de la peau ou des lèvres, plus particulièrement de la peau du visage, en particulier de la peau ridée, comprenant l'application topique sur la peau ou des lèvres d'une composition, notamment cosmétique, comprenant un polymère acrylique tel que décrit précédemment.

L'invention a également pour objet un procédé, notamment cosmétique, de soin de la peau, plus particulièrement de la peau du visage, en particulier de la peau ridée, comprenant l'application topique sur la peau d'une composition, notamment cosmétique, comprenant un polymère acrylique tel que décrit précédemment.

Le procédé selon l'invention est en particulier destiné à lisser la peau humaine du visage et/ou du corps et/ou à diminuer ou effacer les signes du vieillissement cutané, en particulier à réduire ou effacer les rides et/ou les ridules de la peau.

L'invention a également pour objet l'utilisation cosmétique en tant qu'agent tenseur de la peau, en particulier d'une peau ridée, d'un polymère acrylique tel que décrit précédemment, ou d'une composition le contenant et comprenant un milieu physiologiquement acceptable, tel que défini ci-après.

On entend par « agent tenseur » des composés susceptibles d'avoir un effet tenseur apparent, c'est-à-dire de lisser la peau et réduire, voire faire disparaître, de façon immédiate les rides et les ridules.

L'effet tenseur peut être caractérisé par un test *in vitro* de rétractation tel que décrit à l'exemple 4.

Comme exemple de (méth)acrylate d'alkyle en C₆-C₁₆, on peut citer le (méth)acrylate d'hexyle, le (méth)acrylate d'octyle, le (méth)acrylate de 2-éthyl hexyle, le (méth)acrylate de décyle, le (méth)acrylate de lauryle. On utilise de préférence l'acrylate de 2-éthyl hexyle.

De préférence, le polymère selon l'invention comprend, ou consiste en, de l'acrylate d'isobornyle, l'acrylate de 2-éthylhexyle et l'anhydride maléique.

Un polymère particulièrement préféré est le polymère issu de la polymérisation de :
(a) 50 à 80 % en poids, du poids total de monomères, de (méth)acrylate d'isobornyle
(b) 5 à 25 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 50 à 80 % en poids, du poids total de monomères, de (méth)acrylate d'isobornyle
(b) 5 à 15 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 60 à 80 % en poids, du poids total de monomères, de (méth)acrylate d'isobornyle
(b) 5 à 12 % en poids d'anhydride maléique.
(c) 15 à 30 % en poids de monomère acrylate d'alkyle en C₆-C₁₆.

Avantageusement, le polymère selon l'invention consiste en les monomères décrits précédemment.

Avantageusement, le polymère selon l'invention est non ionique.

De préférence, le polymère acrylique selon l'invention a un poids moléculaire moyen en poids allant de 5000 à 1 000 000 g/mole de préférence allant de 10000 à 500 000 g/mole, et préférentiellement allant de 15000 à 350 000 g/mole.

Le poids moléculaire peut notamment être déterminé par chromatographie par exclusion stérique, éluant THF, étalon polystyrène, détecteur refractomètre 2414 de chez WATERS.

Le copolymère peut être un polymère statistique, alterné (bloc), à gradient. De préférence, le copolymère est statistique.

Comme exemple de (méth)acrylate d'alkyle en C₆-C₁₆, on peut citer le (méth)acrylate d'hexyle, le (méth)acrylate d'octyle, le (méth)acrylate de 2-éthyl hexyle, le (méth)acrylate de décyle, le (méth)acrylate de lauryle. On utilise de préférence l'acrylate de 2-éthyl hexyle.

De préférence, le polymère selon l'invention comprend, ou consiste en, de l'acrylate d'isobornyle, l'acrylate de 2-éthylhexyle et l'anhydride maléique.

Un polymère particulièrement préféré est le polymère issu de la polymérisation de :
(a) 50 à 80 % en poids, du poids total de monomères, de (méth)acrylate d'isobornyle
(b) 5 à 30 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 50 à 80 % en poids, du poids total de monomères, de (méth)acrylate d'isobornyle
(b) 5 à 25 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 50 à 80 % en poids, du poids total de monomères, de (méth)acrylate d'isobornyle
(b) 5 à 15 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 60 à 80 % en poids, du poids total de monomères, de (méth)acrylate d'isobornyle
(b) 5 à 12 % en poids d'anhydride maléique.
(c) 15 à 30 % en poids de monomère acrylate d'alkyle en C₆-C₁₆.

Un polymère particulièrement préféré est le polymère issu de la polymérisation de :
(a) 60 à 80 % en poids, du poids total de monomères, de (méth)acrylate d'isobornyle
(b) 5 à 12 % en poids d'anhydride maléique.
(c) 18 à 30 % en poids de monomère acrylate d'alkyle en C₆-C₁₆.

Avantageusement, le polymère selon l'invention consiste en les monomères décrits précédemment.

Avantageusement, le polymère selon l'invention est non ionique.

De préférence, le polymère acrylique selon l'invention a un poids moléculaire moyen en poids allant de 5000 à 1 000 000 g/mole de préférence allant de 10000 à 500 000 g/mole, et préférentiellement allant de 15000 à 350 000 g/mole.

Le poids moléculaire peut notamment être déterminé par chromatographie par exclusion stérique , éluant THF, étalon polystyrène, détecteur refractomètre 2414 de chez WATERS.

Le copolymère peut être un polymère statistique, alterné (bloc), à gradient. De préférence, le copolymère est statistique.

Le copolymère selon l'invention peut être préparé par polymérisation radicalaire des monomères décrits précédemment, notamment en mélange ou ajoutés séquentiellement pendant la polymérisation, notamment en utilisant un solvant organique ayant un point d'ébullition supérieur ou égal à 60 °C, comme par exemple l'isododécane, l'éthanol, l'acétate d'éthyle, le tétrahydrofurane, le méthyltétrahydrofurane, la méthyl éthyl cétone. Le solvant organique permet de solubiliser les monomères mis en oeuvre et le polymère formé.

La polymérisation est notamment effectuée en présence d'un amorceur radicalaire notamment de type peroxyde (par exemple tert-Butyl peroxy-2-ethylhexanoate : Trigonox 21S; 2,5-dimethyl-2,5-di(2-ethylhexanoylperoxy)hexane :Trigonox 141; tert-butyl peroxypivalate : Trigonox 25C75 de chez AkzoNobel) ou azoïque par exemple (AIBN : azobisisobutyronitrile ; V50 : 2,2'-azo-bis(2-amidinopropane) dihydrochlorure).

La polymérisation peut s'effectuer à une température allant de 60 à 100 °C, de préférence allant de 60 à 85 °C.
La durée de la polymérisation peut être d'environ 24 heures.

Le polymère selon l'invention peut être utilisé dans une composition comprenant un milieu physiologiquement acceptable, en particulier dans une composition cosmétique.

Par milieu physiologiquement acceptable, on entend un milieu compatible avec les matières kératiniques d'êtres humains, en particulier avec la peau.

Par composition cosmétique, on entend une composition compatible avec les matières kératiniques, qui présente une couleur, une odeur et un toucher agréables, et qui ne génère pas d'inconforts inacceptables (picotements, tiraillements, rougeurs), susceptibles d'en détourner le consommateur.

Le polymère acrylique tel que défini précédemment peut être présent dans la composition selon l'invention en une teneur allant de 0,1 à 10 % en poids, par rapport au poids total de la composition, de préférence de 0,5 % à 10 % en poids de matière active, et préférentiellement allant de 1 % à 8 % en poids, et plus préférentiellement allant de 1 % à 6 % en poids.

La composition utilisée selon l'invention est généralement adaptée à une application topique sur la peau ou les lèvres et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Selon un mode préféré de réalisation de l'invention, la composition comprenant le polymère peut contenir une huile hydrocarbonée.
L'huile hydrocarbonée est une huile liquide à température ambiante (25 °C).
Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

L'huile hydrocarbonée peut être volatile ou non volatile.

L'huile hydrocarbonée peut être choisie parmi :
les huiles hydrocarbonées ayant de 8 à 14 atomes de carbone, et notamment :
   - les alcanes ramifiés en C₈-C₁₄ comme les isoalcanes en C₈-C₁₄ d'origine pétrolière (appelées aussi isoparaffines) comme l'isododécane (encore appelé 2,2,4,4,6-pentaméthylheptane), l'isodécane, et par exemple les huiles vendues sous les noms commerciaux d'Isopars' ou de Permetyls,
   - les alcanes linéaires, par exemple tels que le n-dodécane (C12) et le n-tétradécane (C14) vendus par Sasol respectivement sous les références PARAFOL 12-97 et PARAFOL 14-97, ainsi que leurs mélanges, le mélange undécane-tridécane, les mélanges de n-undécane (C11) et de n-tridécane (C13) obtenus aux exemples 1 et 2 de la demande WO2008/155059 de la Société Cognis, et leurs mélanges.
les esters à chaîne courte (ayant de 3 à 8 atomes de carbone au total) tels que l'acétate d'éthyle, l'acétate de méthyle, l'acétate de propyle, l'acétate de n-butyle
   - les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
   - les éthers de synthèse ayant de 10 à 40 atomes de carbone ;
   - les hydrocarbures linéaires ou ramifiés, d'origine minérale ou synthétique tels que la vaseline, les polydécènes, le polyisobutène hydrogéné tel que le Parleam ®, le squalane , les huiles de paraffine, et leurs mélanges,
   - les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
   - les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol.

Avantageusement, l'huile hydrocarbonée est apolaire (donc formée uniquement d'atomes de carbone et d'hydrogène).

L'huile hydrocarbonée est de préférence choisie parmi les huiles hydrocarbonées ayant de 8 à 14 atomes de carbone, en particulier les huiles apolaire, décrites précédemment.

Préférentiellement, l'huile hydrocarbonée est l'isododécane.

La composition comprenant le polymère peut contenir, en plus de l'huile hydrocarbonée, une huile siliconée. On entend par « huile siliconée », une huile comprenant au moins un atome de silicium, et notamment au moins un groupe Si-O. L'huile siliconée peut être volatile ou non volatile.

On entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa (10⁻³ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).
On entend par « huile non volatile », une huile ayant une pression de vapeur inférieure à 0,13 Pa.

Comme huiles siliconées volatiles, on peut citer les huiles de silicones linéaires ou cycliques volatiles, notamment, celles ayant une viscosité ≤ 8 centistokes (cSt) (8 x 10⁻⁶ m²/s), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Comme huiles siliconées non volatiles, on peut citer les polydiméthylsiloxanes (PDMS) non volatiles, linéaires ou cycliques; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates.

Avantageusement, la composition peut comprendre une huile hydrocarbonée en un teneur allant de 60 à 100 % en poids du poids total des huiles présentes dans la composition et de 0 à 40 % en poids d'huile siliconée. Selon un mode préféré de l'invention, la composition contient comme huile uniquement une huile hydrocarbonée.

La composition selon l'invention peut comprendre un additif cosmétique choisi parmi l'eau, les parfums, les conservateurs, les charges, les filtres UV, les huiles, les cires, les tensioactifs, les hydratants, les vitamines, les céramides, les antioxydants, les agents anti radicaux libres, les polymères, les épaississants, les matières colorantes.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

Avantageusement, la composition selon l'invention est une composition de soin de la peau.

La composition selon l'invention peut être une composition de maquillage tel qu'un fond de teint, un rouge à lèvres, un liner.

Selon un mode de réalisation, la composition selon l'invention est une composition de maquillage et comprend une huile volatile et une huile non volatile telles que décrites précédemment. En particulier, la composition de maquillage peut comprendre une huile volatile hydrocarbonée et une huile non volatile hydrocarbonée.

Selon un mode de réalisation, la composition selon l'invention est une composition anhydre. On entend par composition anhydre une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.

L'application de la composition cosmétique utilisé selon l'invention se fait selon les techniques habituelles, par exemple par application (notamment de crèmes, de gels, de sérums, de lotions) sur la peau destinée à être traitée, en particulier la peau du visage et/ou du cou, notamment la peau du contour de l'oeil. Dans le cadre de ce procédé, la composition peut être, par exemple, une composition de soin.

L'invention est illustrée plus en détail dans les exemples suivants.

### Exemple 1 : Copolymère acrylate d'isobornyle / acrylate de 2-éthyl hexyle / anhydride maléique (70/20/10 en poids)

Dans un réacteur double enveloppe de 1 litre muni d'une ancre d'agitation on a introduit 70 g d'acrylate d'isobornyle, 20 g d'acrylate de 2-éthyl hexyle et 10 g d'anhydride maléique. Un mélange de 70 g d'isododécane et de 30 g d'acétate d'éthyle a été ensuite ajouté. Le milieu a été porté à une température de 40°C sous agitation (150 tours/min) et un bullage d'argon a été effectué pendant 10 minutes, puis on a ajouté 0.5g d'amorceur tert-Butyl peroxy-2-ethylhexanoateTrigonox 21S (Trigonox® 21S de chez Akzo Nobel)

Le chauffage de la double enveloppe a été réglé sur 90°C pendant 7 heures à 150 tours/min.
Le milieu a ensuite été dilué avec 300 g d'isododécane, puis concentré par distillation pour éliminer l'acétate d'éthyle et l'anhydride maléique qui n'a pas réagi.
On a obtenu au final une solution à 30 % en poids du copolymère dans l'isododécane (rendement supérieur à 90 %).
Le polymère obtenu a un poids moléculaire (Mw) proche de 200 000 g/mol.

### Exemple 2 : Copolymère acrylate d'isobornyle / acrylate de 2-éthyl hexyle / anhydride maléique (65/25/10 en poids)

Le polymère a été préparé selon le mode opératoire de l'exemple 1 en utilisant 65 g d'acrylate d'isobornyle, 25 g d'acrylate de 2-éthyl hexyle et 10 g d'anhydride maléique.

On a obtenu au final une solution à 30 % en poids du copolymère dans l'isododécane (rendement supérieur à 90 %).

Le polymère obtenu a un poids moléculaire (Mw) proche de 200 000g/mol.

### Exemple 3 : Copolymère acrylate d'isobornyle / acrylate de 2-éthyl hexyle / anhydride maléique (75/20/5 en poids)

Le polymère a été préparé selon le mode opératoire de l'exemple 1 en utilisant 75 g d'acrylate d'isobornyle, 20 g d'acrylate de 2-éthyl hexyle et 10 g d'anhydride maléique.

On a obtenu au final une solution à 30 % en poids du copolymère dans l'isododécane (rendement supérieur à 90 %).

Le polymère obtenu a un poids moléculaire (Mw) proche de 200 000g/mol.

### Exemple 4 : Copolymère acrylate d'isobornyle / acrylate de 2-éthyl hexyle / anhydride maléique (60/20/20 en poids)

Le polymère a été préparé selon le mode opératoire de l'exemple 1 en utilisant 60 g d'acrylate d'isobornyle, 20 g d'acrylate de 2-éthyl hexyle et 20 g d'anhydride maléique.

On a obtenu au final une solution à 36 % en poids du copolymère dans l'isododécane (rendement supérieur à 90 %).

Le polymère obtenu a un poids moléculaire (Mw) proche de 200 000 g/mol.

### Exemple 5 :

### Mise en évidence de l'effet tenseur des polymères utilisés selon l'invention

Cet essai consiste à comparer *in vitro* le pouvoir tenseur du polymère à évaluer par rapport à un polymère tenseur de référence : Hybridur® 875 polymer dispersion de chez Air Products (dispersion aqueuse à 40 % en poids de particules d'un réseau interpénétré de polymères polyuréthane et acrylique). Le polymère à évaluer a été déposé sur une bandelette de caoutchouc nitrile découpée dans un gant vendu sous la référence « Safeskin Nitrile Criticial » n° 038846 par la société Dominique Dutscher SA, d'une surface de 3,5 cm² préalablement tendues sur un support. Une solution aqueuse contenant le polymère à évaluer est donc déposée sur la bandelette d'élastomère, en déposant 1,8 mg (en matières sèches) de polymère.

On a déposé ainsi sur une bandelette de caoutchouc nitrile 26 µl d'une solution aqueuse contenant 7 % MA de polymère Hybridur® 875 pour obtenir ainsi une bandelette de référence tenseur et sur une autre bandelette on a déposé 26 µl d'une solution contenant 7 % MA de polymère acrylique à évaluer dans un mélange isododécane/éthanol (70/30 poids/poids).

Après séchage pendant 24 heures à la température ambiante (25 °C), on observé le recourbement (rétractation) de la bandelette traitée avec le polymère acrylique en comparaison avec celui obtenu avec le témoin (Hybridur® 875).

On a mesuré l'effet tenseur obtenu selon le protocole décrit précédemment des polymères des exemples 1 à 3. Puis on a également évalué la résistance à l'eau de l'effet tenseur en plongeant les bandelettes de caoutchouc traitées avec le polymère à évaluer dans l'eau à la température ambiante 25 °C) pendant 10 minutes, puis en évaluant l'effet tenseur après 1 heure de séchage.

On a obtenu les résultats suivants :

| **Polymère testé** | **Effet tenseur** | **Effet tenseur après immersion dans l'eau** |
|---|---|---|
| Référence Hybridure 875 | correct | Correct |
| Exemple 1 | supérieur à la référence | supérieur à la référence |
| Exemple 2 | comparable à la référence | supérieur à la référence |
| Exemple 3 | comparable à la référence | supérieur à la référence |

Les résultats obtenus montrent que les polymères des exemples 1 à 3 forment un film qui présente un bon effet tenseur, y compris après immersion dans l'eau

### Exemple 6 :

On prépare un gel anti-rides ayant la composition suivante :

| | |
|---|---|
| - polymère de l'exemple 1 en solution à 30 % en poids dans l'isododécane | 7 g MA |
| - disteardimonium hectorite/ppropylene carbonate dans isodoecane (bentone gel ® ISDV de chez Elementis) | 3 g |
| - Conservateurs qs | |
| - Isododecane/éthanol (80/20 p/p) qsp | 100 g |

Une composition similaire est également préparée en utilisant le polymère de l'exemple 2 ou 3 ou 4.

La composition obtenue appliquée sur le visage permet de lisser efficacement les rides.

### Exemple 7 à 14 : Evaluation cosmétique de compositions de maquillage

On a préparé les 8 compositions de maquillage selon l'invention décrites ci-dessous.
On a appliqué chaque composition sur un support équivalent de peau en élastomère en réalisant un dépôt d'une épaisseur de 100 µm humide et on a laissé sécher à température ambiante (25 °C) pendant 24 heures.

On a ensuite observé l'état du film obtenu.
On a évalué la résistance à l'eau du film obtenu en appliquant 0,5 ml d'eau ; après 5 minutes de contact on a frotté la surface du film avec un coton puis on a observé l'état du film.
On a également évalué l'aspect collant du film et son aptitude à transférer ou non en touchant le film avec le doigt.

L'évaluation a été faite de la façon suivante :
+++ : propriété cosmétique évaluée très performante
++ : propriété cosmétique évaluée moyennement performante
+ : propriété cosmétique évaluée peu performante
o : propriété cosmétique évaluée non performante

On a obtenu les résultats suivants :

| | Exemple 7 | Exemple 8 | Exemple 9 | Exemple 10 |
|---|---|---|---|---|
| Polymère de l'exemple 1 | 20 g | 20 g | 20 g | 20 g |
| Pâte pigmentaire à 40 % en poids de pigment dans l'iosododécane | 5 g avec DC Red 7 | 5 g avec DC Red 7 | 5 g avec oxyde de fer noir | 5 g avec oxyde de fer noir |
| Disteardimonium Hectorite (BENTONE GEL ISD V de chez Elementis) | 10g | 10g | 10g | 10g |
| Isododécane | 65 g | 45 g | 65 g | 45 g |
| Octyl-2 dodécanol | - | 20 g | - | 20 g |
| Aspect du film | Film homogène | Film homogène | Film homogène | Film homogène |
| Résistance à l'eau | +++ | +++ | +++ | +++ |
| Non collant | +++ | +++ | +++ | +++ |
| Non transfert | +++ | +++ | +++ | +++ |

| | Exemple 11 | Exemple 12 | Exemple 13 | Exemple 14 |
|---|---|---|---|---|
| Polymère de l'exemple 4 | 20 g | 20 g | 20 g | 20 g |
| Pâte pigmentaire à 40 % en poids de pigment dans l'iosododécane | 5 g avec DC Red 7 | 5 g avec DC Red 7 | 5 g avec oxyde de fer noir | 5 g avec oxyde de fer noir |
| Disteardimonium Hectorite (BENTONE GEL ISD V de chez Elementis) | 10g | 10g | 10g | 10g |
| Isododécane | 65 g | 45 g | 65 g | 45g |
| Octyl-2 dodécanol | - | 20 g | - | 20g |
| Aspect du film | Film homogène | Film homogène | Film homogène | Film homogène |
| Résistance à l'eau | +++ | +++ | +++ | +++ |
| Non collant | +++ | +++ | +++ | +++ |
| Non transfert | +++ | +++ | +++ | +++ |

Les résultats obtenus montrent que les polymères 1 et 4 avec ou sans octyl-2 dodécanol forment un film homogène non collant et ne transférant pas au doigt, et résistant à l'eau.

## Revendications

1. Copolymère issu de la polymérisation de :
(a) 50 à 80 % en poids, du poids total de monomères, de (méth)acrylate d'isobornyle
(b) 5 à 30 % en poids d'anhydride maléique
(c) 15 à 30 % en poids de monomère acryiate d'alkyle en C₆-C₁₆.

2. Copolymère selon la revendication précédente, **caractérisé en ce qu'**il est issu de la polymérisation de :
(a) 50 à 80 % en poids, du poids total de monomères, de (méth)acrylate d'isobornyle
(b) 5 à 15 % en poids d'anhydride maléique.
(c) 15 à 30 % en poids de monomère acrylate d'alkyle en C₆-C₁₆,

3. Copolymère selon l'une des revendications précédentes, **caractérisé en ce qu'**il comprend de l'acrylate d'isobornyle, l'acrylate de 2-éthylhexyle et l'anhydride maléique,

4. Copolymère selon l'une quelconque des revendications précédentes, **caractérisé en ce qu'**il a un poids moléculaire moyen en poids allant de 5000 à 1 000 000 g/mole de préférence allant de 10000 à 500 000 g/mole, et préférentiellement allant de 15000 à 350 000 g/mole, déterminé par chromatogaphie par exclusion stérique, éluant THF, étalon polystyrène, détecteur refractomètre 2414 de chez WATERS.

5. Copolymère selon l'une quelconque des revendications précédentes, **caractérisé en ce qu'**il s'agit d'un polymère statistique.

6. Composition comprenant, dans un milieu physiologiquement acceptable, un polymère selon l'une des revendications précédentes.

7. Composition selon la revendication précédente, **caractérisée en ce que** le polymère est présent en une teneur allant de 0,1 à 10 % en poids, par rapport au poids total de la composition, de préférence de 0,5 % à 10 % en poids, et préférentiellement allant de 1 % à 8 % en poids, et plus préférentiellement allant de 1 % à 6 % en poids.

8. Composition selon la revendication 6 ou 7, **caractérisée en ce qu'**elle comprend une huile hydrocarbonée, de préférence une huile hydrocarbonée apolaire ayant de 8 à 14 atomes de carbone, préférentiellement l'isododécane.

9. Composition selon l'une des revendications 6 à 8, **caractérisée en ce qu'**elle comprend un additif choisi l'eau, les parfums, les conservateurs, les charges, les matières colorantes, les filtres UV, les huiles, les cires, les tensioactifs, les hydratants, les vitamines, les céramides, les antioxydants, les agents anti radicaux libres, les polymères, les épaississants, les huiles siliconées, les matières colorantes.

10. Composition selon l'une des revendications 6 à 9, **caractérisée en ce qu'**elle une composition de maquillage comprenant une huile volatile et une huile non volatile, de préférence une huile volatile hydrocarbonée et une huile non volatile hydrocarbonée.

11. Composition selon l'une quelconque des revendications 6 à 10, **caractérisée en ce qu'**elle est anhydre.

12. Procédé cosmétique de soin ou de maquillage de la peau ou des lèvres, plus particulièrement de la peau du visage, en particulier de la peau ridée, comprenant l'application topique sur la peau ou les lèvres d'une composition selon l'une des revendications 6 à 11.

13. Procédé selon la revendication précédente, **caractérisé en ce qu'**il s'agit d'un procédé cosmétique de soin de la peau plus particulièrement de la peau du visage, en particulier de la peau ridée avec l'application topique sur la peau de ladite composition.

14. Procédé selon la revendication précédente, **caractérisé en ce qu'**il est destiné à atténuer les rides.

15. Utilisation cosmétique en tant qu'agent tenseur de la peau, en particulier d'une peau ridée, du polymère tel que défini dans l'une quelconque des revendications 1 à 5.

## Patentansprüche

1. Copolymer aus der Polymerisation von:
(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% C₆-G₁₆-Alkylacrylat-Monomer.

2. Copolymer nach dem vorhergehenden Anspruch, **dadurch gekennzeichnet, dass** es aus der Polymerisation von:
(a) 50 bis 80 Gew.-%, bezogen auf das Gesamtgewicht der Monomere, Isobornyl(meth)acrylat,
(b) 5 bis 15 Gew.-% Maleinsäureanhydrid,
(c) 15 bis 30 Gew.-% C₆-C₁₆-Alkylacrylat-Monomer stammt.

3. Copolymer nach einem der vorhergehenden Ansprüche, **dadurch gekennzeichnet, dass** es Isobornylacrylat, 2-Ethylhexylacrylat und Maleinsäureanhydrid umfasst.

4. Copolymer nach einem der vorhergehenden Ansprüche, **dadurch gekennzeichnet, dass** es ein durch Größenausschlusschromatographie, Elutionsmittel THF, Polystyrol-Standard, Detektor Refraktometer 2414 von Waters, bestimmtes gewichtsmittleres Molekulargewicht im Bereich von 5000 bis 1.000.000 g/mol, bevorzugt im Bereich von 10.000 bis 500.000 g/mol und vorzugsweise im Bereich von 15.000 bis 350.000 g/mol aufweist.

5. Copolymer nach einem der vorhergehenden Ansprüche, **dadurch gekennzeichnet, dass** es sich um ein statistisches Polymer handelt.

6. Zusammensetzung, die in einem physiologisch unbedenklichen Medium ein Polymer nach einem der vorhergehenden Ansprüche umfasst.

7. Zusammensetzung nach dem vorhergehenden Anspruch, **dadurch gekennzeichnet, dass** das Polymer in einem Gehalt im Bereich von 0,1 bis 10 Gew.-%, bezogen auf das Gesamtgewicht der Zusammensetzung, bevorzugt 0,5 bis 10 Gew.-% und vorzugsweise im Bereich von 1 bis 8 Gew.-% und weiter bevorzugt im Bereich von 1 bis 6 Gew.-% vorliegt.

8. Zusammensetzung nach Anspruch 6 oder 7, **dadurch gekennzeichnet, dass** sie ein Kohlenwasserstofföl, bevorzugt ein unpolares Kohlenwasserstofföl mit 8 bis 14 Kohlenstoffatomen, vorzugsweise Isododecan, umfasst.

9. Zusammensetzung nach einem der Ansprüche 6 bis 8, **dadurch gekennzeichnet, dass** sie ein Additiv umfasst, das aus Wasser, Duftstoffen, Konservierungsstoffen, Füllstoffen, Farbmitteln, UV-Filtern, Ölen, Wachsen, Tensiden, Feuchtigkeitsmitteln, Vitaminen, Ceramiden, Antioxidantien, Mitteln gegen freie Radikale, Polymeren, Verdickungsmitteln, Silikonölen und Farbmitteln ausgewählt ist.

10. Zusammensetzung nach einem der Ansprüche 6 bis 9, **dadurch gekennzeichnet, dass** es sich um eine Schminkzusammensetzung handelt, die ein flüchtiges Öl und ein nichtflüchtiges Ö1, bevorzugt ein flüchtiges Kohlenwasserstofföl und ein nichtflüchtiges Kohlenwasserstofföl, umfasst.

11. Zusammensetzung nach einem der Ansprüche 6 bis 10, **dadurch gekennzeichnet, dass** sie wasserfrei ist.

12. Kosmetisches Verfahren zur Pflege oder zum Schminken der Haut oder der Lippen, insbesondere der Gesichtshaut, insbesondere von faltiger Haut, umfassend das topische Aufbringen einer Zusammensetzung nach einem der Ansprüche 6 bis 11 auf die Haut oder die Lippen.

13. Verfahren nach dem vorhergehenden Anspruch, **dadurch gekennzeichnet, dass** es sich um ein kosmetisches Verfahren zur Pflege der Haut, spezieller der Gesichtshaut, insbesondere faltiger Haut, mit topischem Aufbringen der Zusammensetzung auf die Haut handelt.

14. Verfahren nach dem vorhergehenden Anspruch, **dadurch gekennzeichnet, dass** es zur Abschwächung von Falten vorgesehen ist.

15. Kosmetische Verwendung des Polymers gemäß einem der Ansprüche 1 bis 15 als Straffungsmittel für die Haut, insbesondere für faltige Haut.

## Claims

1. Copolymer resulting from the polymerization of:
(a) 50% to 80% by weight, of the total weight of monomers, of isobornyl (meth)acrylate,
(b) 5% to 30% by weight of maleic anhydride,
(c) 15% to 30% by weight of C6-C16 alkyl acrylate monomer.

2. Copolymer according to the preceding claim, **characterized in that** it results from the polymerization of:
(a) 50% to 80% by weight, of the total weight of monomers, of isobornyl (meth)acrylate,
(b) 5% to 15% by weight of maleic anhydride,
(c) 15% to 30% by weight of C6-C16 alkyl acrylate monomer.

3. Copolymer according to either of the preceding claims, **characterized in that** it comprises isobornyl acrylate, 2-ethylhexyl acrylate and maleic anhydride.

4. Copolymer according to any one of the preceding claims, **characterized in that** it has a weight-average molecular weight ranging from 5000 to 1 000 000 g/mol, preferably ranging from 10 000 to 500 000 g/mol and preferentially ranging from 15 000 to 350 000 g/mol, determined by size exclusion chromatography, eluent THF, polystyrene standard, Waters 2414 refractive index detector.

5. Copolymer according to any one of the preceding claims, **characterized in that** it is a random polymer.

6. Composition comprising, in a physiologically acceptable medium, a polymer according to one of the preceding claims.

7. Composition according to the preceding claim, **characterized in that** the polymer is present in a content ranging from 0.1% to 10% by weight, relative to the total weight of the composition, preferably from 0.5% to 10% by weight, preferentially ranging from 1% to 8% by weight and more preferentially ranging from 1% to 6% by weight.

8. Composition according to Claim 6 or 7, **characterized in that** it comprises a hydrocarbon oil, preferably a nonpolar hydrocarbon oil having from 8 to 14 carbon atoms, preferentially isododecane.

9. Composition according to one of Claims 6 to 8, **characterized in that** it comprises an additive chosen from water, fragrances, preservatives, fillers, colorants, UV-screening agents, oils, waxes, surfactants, moisturizing agents, vitamins, ceramides, antioxidants, agents for combating free radicals, polymers, thickeners, silicone oils and colorants.

10. Composition according to one of Claims 6 to 9, **characterized in that** it is a makeup composition comprising a volatile oil and a nonvolatile oil, preferably a volatile hydrocarbon oil and a nonvolatile hydrocarbon oil.

11. Composition according to any one of Claims 6 to 10, **characterized in that** it is anhydrous.

12. Cosmetic method for caring for or making up the skin or the lips, more particularly the skin of the face, in particular wrinkled skin, comprising the topical application to the skin or the lips of a composition according to one of Claims 6 to 11.

13. Method according to the preceding claim, **characterized in that** it is a cosmetic method for caring for the skin, more particularly for the skin of the face, in particular for wrinkled skin, with the topical application to the skin of said composition.

14. Method according to the preceding claim, **characterized in that** it is intended to attenuate wrinkles.

15. Cosmetic use, as tightening agent for the skin, in particular for wrinkled skin, of the polymer as defined in any one of Claims 1 to 5.
